# EUROPÄISCHE PATENTSCHRIFT

(11) **EP 2 664 281 B1**
(45) Veröffentlichungstag und Bekanntmachung des Hinweises auf die Patenterteilung: **16.09.2015**
(21) Anmeldenummer: 13167497.0
(22) Anmeldetag: 13.05.2013
(51) Int. Cl.: A61B 17/02, A61B 19/00

(54) **Klammer für eine Wundspreizvorrichtung**
Clamp for a surgical wound retractor
Pince pour un dispositif de rétracteur chirugical

(30) Priorität: 14.05.2012 DE 102012104179
(43) Veröffentlichungstag der Anmeldung: 20.11.2013
(73) Patentinhaber: Ceatec Medizintechnik GmbH, 78573 Wurmlingen (DE); Unger, Michael, 78573 Wurmlingen (DE)
(72) Erfinder: Unger, Michael, 78573 Wurmlingen (DE)
(74) Vertreter: Patentanwälte und Rechtsanwalt Weiß, Arat & Partner mbB

(56) Entgegenhaltungen:
- WO-A2-2007/022159
- US-A1- 2005 080 321
- US-A1- 2005 131 394
- US-A1- 2007 191 686

## Beschreibung

Die Erfindung betrifft eine Klammer für eine Wundspreizeinrichtung nach dem Oberbegriff des Anspruchs 1.

### STAND DER TECHNIK

Aus dem Stand der Technik sind verschiedene Klammern für Wundspreizvorrichtungen bekannt und gebräuchlich. Nachteilig an diesen Klammern ist allerdings der Umstand, dass sie sehr umständlich aufgebracht und entsprechend wieder abgezogen werden müssen. Ein einhändiges Aufbringen solcher Klammern ist fast nicht möglich.
In diesem Zusammenhang wird auf die US 2005/080321 A1 verwiesen. Dort ist eine Klammer für eine Wundspreizvorrichtung gezeigt. Diese Wundspreizvorrichtung wird dadurch festgelegt, dass ein schwenkbar angeordnetes Teil gegen eine Wandung der Vorrichtung klemmend verschwenkbar ist. Ausserdem wird auf die US 2007/191686 A1 hingewiesen, welche eine Klammer zeigt, bei der zwei Hälften eines einstückigen Teils, welche zueinander spreizbar sind und somit festgelegt werden. Weiter wird auf die US 2005/131394 A1 hingewiesen, welche einen Stempel zeigt, welcher zur Aufnahme eines Krankenbettgestänges geeignet ist und durch Einziehen in die gezeigte Vorrichtung eine Klemmung des eingelegten Krankenbettgestänges erreicht. Daneben wird auf die WO 2007/022159 A1 verwiesen, bei der eine Festlegung der Klammer durch ein gegen eine Federkraft öffnebare Schublade erfolgt, wobei die Schublade nach Aufbringen auf das Krankenbettgestänge zurückschnappt und die Klammer festlegt.

Aus dem Stand der Technik sind verschiedene Klammern bekannt. In diesem Zusammenhang wird auf die DE 197 28 741 C1 hingewiesen. Dort ist eine Schnellspannklammer für eine Wundspreizvorrichtung mit einer Aufnahme für einen Arm eines Wundspreizhakens und einem Halter zur Befestigung auf einem Arm eines Wundspreizrahmens offenbart. In diesem Zusammenhang wird ein Halter aus einer Federklammer beschrieben, bei dem der maximale Innendurchmesser einer Ausnehmung im unverspannten Zustand gleich oder geringfügig kleiner oder grösser ist als der Durchmesser eines Arms eine Wundspreizrahmens und die maulförmige Öffnung elastisch bis auf den Durchmesser eines Armes eines Wundspreizrahmens aufweitbar ist, wobei die Federklammer aus einem Klammeroberteil und einem Klammerunterteil besteht und Klammeroberteil und Klammerunterteil über eine Gelenkverbindung miteinander verbunden sind und an mindestens einem der beiden Klammerteile eine Federlasche angeordnet ist, die sich am anderen Klammerteil und mindestens in einem aufgeweiteten Zustand während der Montage der Schnellspannklammer an einem Wundspreizrahmen in der Federklammer eine Schliesskraft erzeugt.
Eine weitere aus dem Stand der Technik bekannte Schnellspannklammer ist in der DE 102 54 006 B3 beschrieben. Dort wird eine Schnellspannklammer mit einem Klammerüberteil mit einer Aufnahme für den Arm eines Instrumentes, einem Klammerunterteil mit einer Aufnahme mit einer maulförmigen Öffnung zur Anordnung der Schnellspannklammer an einem zweiten Arm und mit einem das Klammeroberteil und das Klammerunterteil durchgreifenden Spannbolzen zur Verfügung gestellt, die bei normaler Spannkraft eine hohe Haltekraft erzeugt oder bei der eine geringe Spannkraft zu einer ausreichenden Haltekraft der Schnellspannklammer führt, was dadurch erreicht wird, dass das Klammerunterteil als Federzange aus über ein Gelenk gegeneinander bewegbarem Zangenoberteil und Zangenunterteil mit jeweils einem Hebelarm und einem Zangenteil ausgebildet ist, der Spannbolzen durch die der Aufnahme entgegengesetzter Hebelarme geführt ist und das Gelenk zwischen den Hebelarmen und den Zangenteilen angeordnet ist und die Zangenteile die Aufnahme bilden, die den zweiten Arm teilweise umgreifen.

US 2005/0080321 A1 offenbart eine Klammer entsprechend des Oberbegriffs von Anspruch 1.

### Aufgabe der Erfindung

Aufgabe der Erfindung ist es eine Klammer zu schaffen, welche eine einfache und schnelle Möglichkeit zur Verfügung stellt eine Verbindung mit einem Wundspreizrahmen herzustellen. Hierbei soll es insbesondere ermöglicht werden, dass einhändiges Aufklippsen der Klammer durch den Nutzer durchführbar ist.

### Lösung der Aufgabe

Zur Lösung der Aufgabe führen die Merkmale des Anspruchs 1.

Eine erfindungsgemässe Klammer wird für eine Wundspreizvorrichtung eingesetzt. Als Wundspreizvorrichtung werden diejenigen Vorrichtungen bezeichnet, die eine Befestigungsstange aufweisen, an der ein oder mehrere Haltearme angeordnet sind, wobei einer der Halterarme mit einem weiteren Arm versehen ist und alle Arme gemeinsam einen Wundspreizrahmen aufspannen. Die Arme zur Befestigung von Wundspreizern werden auch Retraktoren genannt. Die erfindungsgemässe Klammer ist zur Anbringung auf diesen Armen bzw. Retraktoren vorgesehen.

Eine erfindungsgemässe Klammer zur Anbringung an einem Arm einer Wundspreizvorrichtung weist ausserdem zwei Klemmbacken auf. Diese Klemmbacken sind erforderlich, um den Freibereich der wannenförmigen Ausnehmung zur Aufnahme des Arms zu verringern. Hierzu greifen die Klemmbacken seitlich in den Bereich der wannenförmigen Ausnehmung ein und verringern beispielsweise den Umfang der wannenförmigen Ausnehmung, sodass ein Arm, welcher in der wannenförmigen Ausnehmung eingelegt ist an der erfindungsgemässen Klammer festgehalten wird. Dazu greifen die zwei Klemmbacken jeweils beispielsweise tangential in die wannenförmige Ausnehmung ein. In welcher Weise der Eingriff im Einzelnen von Statten geht, ist durch die Form, Grösse und Ausbildung der Klemmbacken bestimmt. Durch den Einsatz von zwei Klemmbacken ist ein möglichst hohes Druckmoment möglich. Dadurch wird eine besonders feste Klemmung des Arms an der erfindungsgemässen Klammer erreicht.

Eine erfindungsgemässe Klammer weist ausserdem einen Spannbolzen auf. Dieser Spannbolzen ist derart gestaltet, dass er einends eine Klemmbackenaufnahme umfasst und andernends einen Spannbolzenkopf aufweist. Der Begriff des Umfassens bedeutet in diesem Zusammenhang, dass der Spannbolzen entweder fest oder lösbar mit der Klemmbackenaufnahme verbunden ist. Eine feste Verbindung wäre beispielsweise dann gegeben, wenn ein Schweissen oder Verkleben durchgeführt werden würde. Eine lösbare Verbindung wäre dann gegeben, wenn beispielsweise durch eine Schraube und ein dazugehöriges Gewinde im Gegenstück eine Verbindung hergestellt werden würde, die auf einfache Art und Weise durch das Herausdrehen der Schraube mit Werkzeugen oder manuell gelöst werden kann. Vom Begriff des Umfasstseins ist grundsätzlich aber auch die einstückige Ausbildung des Spannbolzens mit Klemmbackenaufnahme umfasst.

Der Spannbolzenkopf ist einends des Spannbolzens angebracht. Andernends des Spannbolzens soll erfindungsgemäss eine wannenförmige Ausnehmung zur Aufnahme des Arms der Wundspreizvorrichtung vorhanden sein. Diese wannenförmige Ausnehmung ist hierbei andernends in der Verlängerung des Spannbolzens angeordnet. Dies bedeutet im Einzelnen, dass die wannenförmige Ausnehmung direkt unterhalb des Spannbolzens oder im Bereich des Spannbolzens am unteren Ende angeordnet ist. Unten oder unterhalb soll hierbei andernends des Spannbolzenkopfs bedeuten. In der Verlängerung des Spannbolzens bedeutet daneben, dass zur Ermittlung der Verlängerung des Spannbolzens der wesentliche Teil des Verlaufs des Spannbolzens berücksichtigt wird. Der wesentliche Teil des Spannbolzens ist in der Regel der Spannbolzen selbst und nicht angeformte oder verbundene oder einstückig mithergestellte Teile wie beispielsweise die Klemmbackenaufnahme. Vorteilhaft an der zentralen Anbringung der wannenförmigen Ausnehmung zur Aufnahme des Arms der Wundspreizvorrichtung direkt unterhalb des Spannbolzens führt zu einem sehr austarierten Verhältnis der einzelnen Teile, sodass ein einhändiges Aufbringen der erfindungsgemässen Klammer auf einen entsprechenden Arm ohne weiteres möglich ist. Nach dem Aufbringen der erfindungsgemässen Klammer kommt es ausserdem nicht zu dem Umstand, dass die Klammer aufgrund von seitlich des Arms befindlichen Schwerpunkten wegklappt, wie dies beispielsweise in den Klammern aus dem Stand der Technik bekannt ist.

Bei einer erfindungsgemässen Klammer kommen grundsätzlich zwei Klemmbacken zum Einsatz. Diese zwei Klemmbacken befinden sich andernends des Spannbolzens. Andernends bedeutet hierbei, dass sich die Klemmbacken andernends des Spannbolzenkopfs befinden. In anderen Worten sind die Klemmbacken am unteren Ende des Spannbolzens angeordnet. Dort dienen sie dazu die wannenförmige Ausnehmung zur Aufnahme des Armes der Wundspreizvorrichtung je nach Wunsch des Nutzer mehr oder weniger fest zu klemmen.

Weiterhin sind die zwei Klemmbacken jeweils exzentrisch drehbar an der Klemmbackenaufnahme gelagert. Die Ausgestaltung der jeweiligen Ausführungsbeispiele sind hierbei zu vernachlässigen, solange eine exzentrische Lagerung der Klemmbacken in der Klemmbackenaufnahme gegeben ist. Durch die exzentrisch drehbare Lagerung wird vorteilhaft erreicht, dass mögliche Hebelwirkungen ausgenutzt werden und dadurch relativ wenig Kraft notwendig ist, um ein optimales Ergebnis zu erzielen. Wenn im Rahmen der exzentrischen drehbaren Lagerung der Klemmbacken an der Klemmbackenaufnahme gesprochen wird, so ist davon auch das exzentrisch schwenkbare Lagern der Klemmbacken in der Klemmbackenaufnahme abgedeckt.

Weiterhin weist die erfindungsgemässe Klammer einen Kraftüberträger auf. Dieser Kraftüberträger ist an dem Spannbolzen gleitbar angeordnet. Dies bedeutet, dass der Kraftüberträger den Spannbolzen in der Regel umschliesst und in dem Bereich zwischen Spannbolzenkopf und Klemmbackenaufnahme hin und her gleiten kann. Die Bewegung des Kraftüberträgers an dem Spannbolzen wird einerseits durch die Klemmbackenaufnahme sowie durch die Klemmbacken selbst begrenzt und andererseits durch eine Klemmeinrichtung begrenzt.

Die Verbindungsstelle zwischen der Klemmeinrichtung und dem Kraftüberträger ist derart gestaltet, dass der Kraftüberträger eine konische Verjüngung hin zum Spannbolzenkopf aufweist. Diese konische Verjüngung greift wiederum in eine Aufnahme der Klemmeinrichtung ein. Dabei ist die konische Verjüngung in die Aufnahme anschlagend verschieblich aufgenommen. Dass bedeutet, dass die konische Verjüngung über einen möglichst grossen Bereich mit der Aufnahme in Kontakt steht. Bei einer Krafteinwirkung vom Spannbolzenkopf hin zu Klemmbackenaufnahme schiebt die Klemmeinrichtung über die Aufnahme den Kraftüberträger nach unten zu den Klemmbacken hin. Dabei erleichtert das Zusammenspiel der konischen Verjüngung und der Aufnahme die Weitergabe der Druckkraft.

Der Kraftüberträger ist im erfindungsgemässen Ausführungsbeispiel derart gestaltet, dass er gegenüber den zumindest zwei Klemmbacken jeweils ein elastisches Mittel, wie beispielsweise eine Feder, aufweist. Der Kraftüberträger ist federgelagert gegenüber den zwei Klemmbacken. Als elastisches Mittel kommt neben der Feder auch ein Gummipuffer oder ein anderes kraftaufnehmendes und wieder abgebendes Element in Betracht. Diese Federlagerung ist notwendig um ein Freigeben eines Arms zu ermöglichen, wenn beispielsweise die Kraft vom Spannbolzenkopf hin zur Klemmbackenaufnahme entfällt. In einem solchen Fall, soll der Kraftüberträger wieder hin zu dem Spannbolzenkopf verschoben werden. Hierzu bedarf es der elastischen Mittel, wie beispielsweise der Feder oder dem Gummipuffer. Dies hat den Vorteil, dass auf einfache Art und Weise eine Reversibilität der aufgebrachten Druckkraft erreicht wird.

In einem erfindungsgemässen Ausführungsbeispiel umfasst der Spannbolzen einen Spannhebel. Dieser Spannhebel ist derart gestaltet, dass er im Wesentlichen halbkreisförmig über dem Spannbolzen geschwenkt werden kann. Wenn der Spannhebel im Wesentlichen im rechten Winkel zu dem Spannbolzen steht, ist aufgrund der Ausbildung des Spannhebels kein Druck auf die Klemmeinrichtung und damit auf den Kraftüberträger und damit auf die Klemmbacken vorhanden. In dem erfindungsgemässen Ausführungsbeispiel entsteht ein solcher Druck erst, wenn der Spannhebel im Wesentlichen die Verlängerung des Spannbolzens nach oben bildet. In anderen Ausführungsbeispielen ist auch eine andere Gestaltung möglich. Es soll lediglich gewährleistet sein, dass durch den Spannhebel ein Druck gegenüber der Klemmeinrichtung erreichbar ist, welcher durch weiteres Umlegen des Spannhebels wieder weggenommen werden kann.

Ausserdem weist die erfindungsgemässe Klammer eine Anordnung der Klemmbacken derart auf, dass die eine Klemmbacke einerseits der wannenförmigen Ausnehmung und die andere Klemmbacke andererseits der wannenförmigen Ausnehmung angeordnet ist. Hierbei dient der Spannbolzen als zentrales Trennelement der beiden Klemmbolzen voneinander. Hierdurch wird vorteilhaft erreicht, dass ein einfaches Austarieren der Klammer möglich wird.

Die erfindungsgemässe Klammer ist derart ausgestaltet, dass der Kraftüberträger andernseits der Klemmbacken mit der Klemmeinrichtung in Wirkverbindung steht. Dies bedeutet im Einzelnen, dass die Klemmeinrichtung die von dem Spannbolzenkopf hin zu den Klemmbacken aufgebrachte Druckkraft an den Kraftüberträger weitergibt, damit dieser wiederum die Druckkraft an die Klemmbacken weitergibt. Auf diese Weise wird eine möglichst reibungsarme Weitergabe der Druckkraft vom Spannbolzenkopf zu den Klemmbacken erreicht. In gleicher Weise umfasst der Begriff der Wirkverbindung die Reversion dieser Druckkraft, in dem die elastischen Mittel, wie beispielsweise Federn oder Gummipuffer den Kraftüberträger hin zum Spannbolzenkopf verschieben und der Kraftüberträger diese Kraft dann wiederum über die Klemmeinrichtung an den Griff weitergegeben wird. Bei einem solchen Vorgang wird nicht nur der Arm in der wannenförmigen Ausnehmung festgelegt, sondern auch gleichzeitig ein in die Klemmeinrichtung eingebrachter Wundspreizer.

In einem erfindungsgemässen Ausführungsbeispiel steht die Klemmeinrichtung mit dem Spannhebel in Wirkverbindung. Dabei weist der Spannhebel und der Spannbolzen einen gemeinsamen Drehpunkt auf, welcher beispielsweise als Achse ausgebildet sein kann. Vorteilhaft hierbei ist eine einfache und schnelle Weitergabe der erzeugten Druckkraft an die Klemmbacken.

### Figurenbeschreibung

Weitere Vorteile, Merkmale und Einzelheiten der Erfindung ergeben sich aus der nachfolgenden Figurenbeschreibung. So zeigt
Figur 1 eine erfindungsgemässe Klammer von schräg oben;
Figur 2 eine geschnittene vergrösserte Seitenansicht der Figur 1.

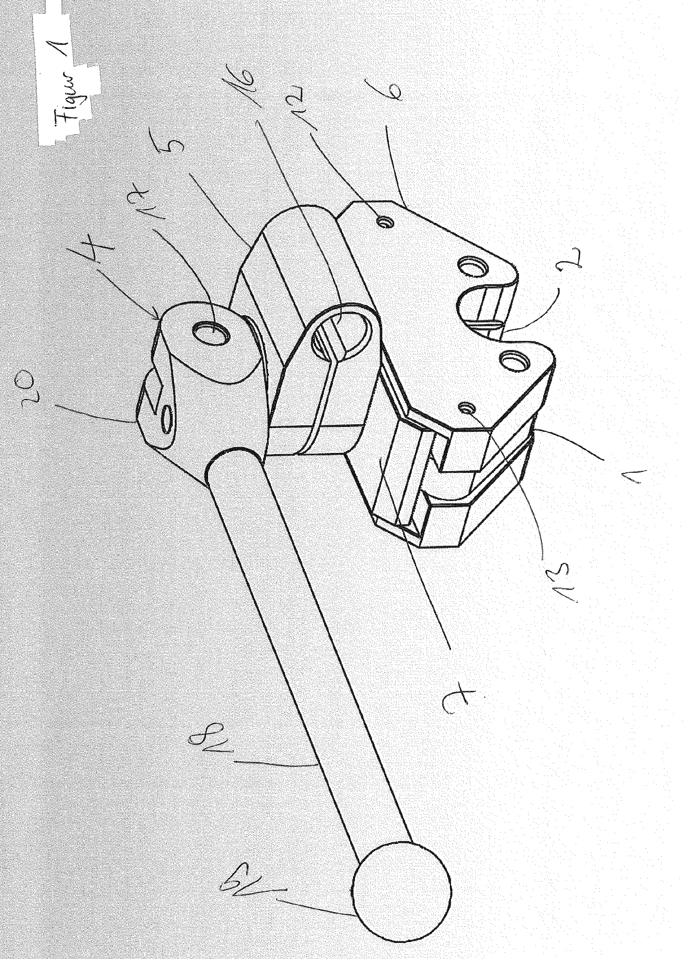

In Figur 1 ist die erfindungsgemässe Klammer gezeigt. Dort ist eine Klemmbackenaufnahme 6 gegeben. In der Klemmbackenaufnahme 6 ist eine Klemmbacke 1 und eine andere Klemmbacke 2 gezeigt, wobei die Klemmbacke 1 über einen Drehpunkt 13 und die andere Klemmbacke 2 über einen Drehpunkt 12 mit der Klemmbackenaufnahme 6 verbunden ist. Daneben ist in Figur 1 zu erkennen, wie ein Kraftüberträger 7 vorhanden ist. Der Kraftüberträger 7 ist in diesem Ausführungsbeispiel im wesentlichen in die Klemmbackenaufnahme 6 aufgenommen. Der Kraftüberträger 7 bildet das Verbindungsstück zwischen der Klemmbackenaufnahme 6 und einer Klemmeinrichtung 5.

Die Klemmeinrichtung 5 weist eine durchgehende Öffnung 16 auf, in welche ein nicht gezeigtes Rohr für ein Werkzeug oder Instrument, wie beispielsweise ein Wundspreizer, eingebracht werden kann.

Ausserdem ist in Figur 1 ein Spannhebel 4 gezeigt, welche über eine Drehachse 17 mit einem in Figur 1 nicht sichtbaren Spannbolzen 3 verbunden ist. Der Spannhebel 4 besteht aus einem Griff 18, einem Halteknauf 19 und ein die Drehachse 17 aufnehmendes Verbindungselement 20.

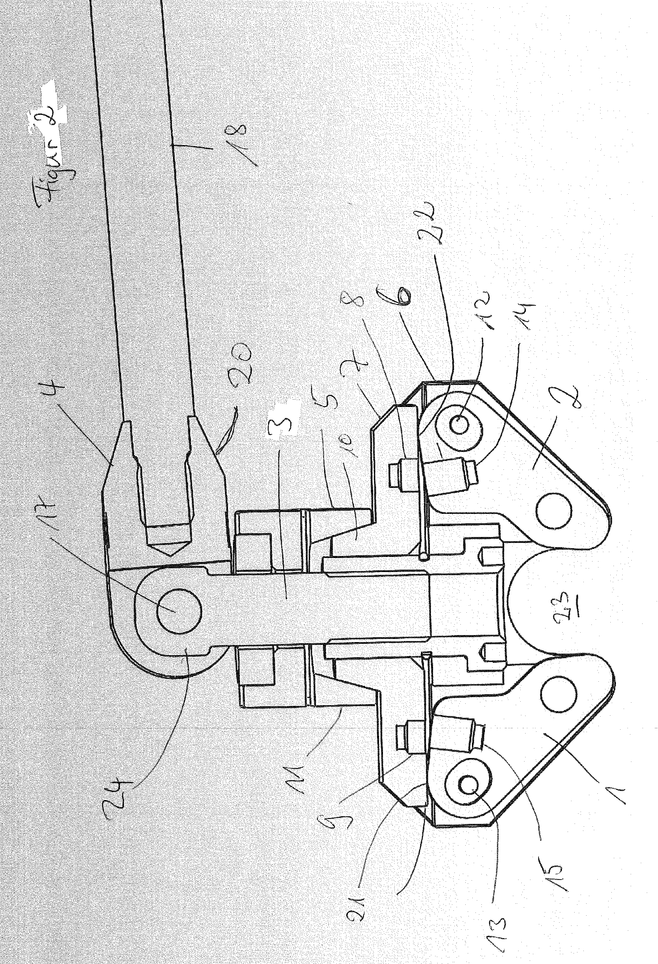

In Figur 2 ist eine geschnittene Seitenansicht eines Teils der Figur 1 gezeigt. Die geschnittene Seitenansicht ist hierbei vergrössert dargestellt. In der geschnittenen Seitenansicht der Figur 2 ist gut zu erkennen, wie die Klemmbacke 1 und die andere Klemmbacke 2 innerhalb der Klemmbackenaufnahme 6 gelagert sind. Die Klemmbacke 1 umfasst einen Drehpunkt 13 und eine Federtasche 15. Die andere Klemmbacke 2 umfasst einen Drehpunkt 12 und eine Federtasche 14. Die Drehpunkte 12, 13 sind als eine gemeinsame Achse ausgeführt, wobei die Achsenden jeweils in die Klemmbackenaufnahme 6 einmünden.

Der Drehpunkt 13 ist exzentrisch an der Klemmbacke 1 angebracht. In gleicher Weise ist der Drehpunkt 12 exzentrisch an der anderen Klemmbacke 2 eingebracht. Die Klemmbacke 1 verfügt über einen Anschlagpunkt 21. Die Klemmbacke 1 steht mit dem Kraftüberträger 7 in Wirkverbindung. Dies bedeutet im Einzelnen, dass der Kraftüberträger 7 bei einer Bewegung hin zu der Klemmbacke 1 zunächst auf den Anschlagpunkt 21 drückt und durch die exzentrische Anbringung des Drehpunkts 13 bewirkt, dass die Klemmbacke 1 in eine wannenförmige Ausnehmung 23 der Klemmbackenaufnahme 6 einragt und somit eine Verringerung des Durchmessers der wannenförmigen Ausnehmung 23 erreicht.

In gleicher Weise verfügt die andere Klemmbacke 2 über einen Anschlagpunkt 22. Bei einer Bewegung des Kraftüberträgers 7 hin zu der anderen Klemmbacke 2 wird über den exzentrisch angebrachten Drehpunkt 12 bewirkt, dass die andere Klemmbacke 2 in die wannenförmige Ausnehmung 23 hineinragt und somit den Freibereich der wannenförmigen Ausnehmung 23 verringert. Die wannenförmige Ausnehmung 23 ist geeignet zur Aufnahme eines nicht gezeigten Arms einer Wundspreizvorrichtung. Diese rohrförmigen Arme dienen dabei der Befestigung von Instrumenten oder Werkzeugen, welche für den Operateur an einer bestimmten Position fixiert werden müssen.

Die erfindungsgemässe Klammer kann hierbei auf einfache Art und Weise direkt auf das Rohrelement aufgeklippst werden. Hierzu ist das Zusammenspiel verschiedener Elemente notwendig. Besonders vorteilhaft ist hierbei der Umstand, dass die wannenförmige Ausnehmung 23 zur Aufnahme des Arms direkt unterhalb des Spannbolzens 3 angeordnet ist. Am oberen Ende des Spannbolzens 3 befindet sich der Spannbolzenkopf 24. Unterhalb des Spannbolzens 3 bedeutet hierbei, dass die wannenförmige Ausnehmung 23 andernends des Spannbolzenkopfs 24 in der Verlängerung des Spannbolzens 3 angeordnet ist.

Dies erleichtert dem Nutzer das einfache Aufklippsen der erfindungsgemässen Klammer auf einen entsprechenden Arm eines Wundspreizrahmens.

Der Kraftüberträger 7 weist zwei Federtaschen 8, 9 auf. Die Federtasche 9 des Kraftüberträgers 7 steht hierbei in Wirkverbindung mit der Federtasche 15 der Klemmbacke 1. Die Federtasche 8 des Kraftüberträgers 7 steht in Wirkverbindung mit der Federtasche 14 der anderen Klemmbacke 2. Die Wirkverbindung ist derart gestaltet, dass zwischen der Federtasche 9 und der Federtasche 15 ein nicht näher gezeigtes Federelement angeordnet ist. In gleicher Weise ist auch in der Federtasche 8 des Kraftüberträgers 7 und der Federtasche 14 der anderen Klemmbacke 2 ein nicht näher gezeigtes Federelement vorhanden. Die Federelemente in den Federtaschen 8, 14 und 9, 15 bewirken, dass die Klemmbacken 1, 2 zu dem Kraftüberträger 7 eine vordefinierte Position einnehmen. Dies kann beispielsweise so eingestellt werden, dass der Nutzer einhändig die erfindungsgemässe Klammer aufklippsen kann und die Klemmbacken 1, 2 derart federgelagert sind, dass die Klammer ohne weiteres Zutun des Nutzers an der aufgeklippsten Position verharrt und nicht von dem Arm des Wundspreizrahmens fällt.

Folglich ist es in dieser vordefinierten Position für den Nutzer der erfindungsgemässen Klammer möglich, dass die Klammer ohne grösseren Kraftaufwand auf einen Arm aufgeklippst werden kann. Die Federelemente sind derart eingestellt, als dass sie um die jeweiligen Drehpunkte 12, 13 der Klemmbacken 1, 2 kurz nachgeben und dann wieder in die vorgegebene Position zurückrutschen, so dass ein Aufklippsen ermöglicht wird.

Der Kraftüberträger 7 weist andernends der Klemmbacken 1, 2 eine konische Verjüngung 10 auf. Die konische Verjüngung 10 greift in eine Aufnahme 11 der Klemmeinrichtung 5 ein. Der Kraftüberträger 7 ist grundsätzlich freigleitend an dem Spannbolzen 3 angeordnet.

In gleicher Weise ist die Klemmeinrichtung 5 freigleitend an dem Spannbolzen 3 angeordnet. Der Spannbolzen 3 ist einends fest mit der Klemmbackenaufnahme 6 verbunden und andernseits von dem Spannhebel 4 begrenzt. Innerhalb des Bereichs zwischen dem Spannhebel 4 und der Klemmbackenaufnahme 6 ist der Kraftüberträger 7 und die Klemmeinrichtung 5 grundsätzlich entlang des Spannbolzens 3 beweglich angeordnet.

In den Figuren 1 und 2 ist die erfindungsgemässe Klammer in einem sogenannten entspannten Zustand dargestellt. Das bedeutet, dass der Spannhebel 4 im Zusammenspiel mit dem Spannbolzen 3 keinen Druck auf die Klemmeinrichtung 5 und somit auf den Kraftüberträger 7 ausübt.

Beim Drehen des Griffs 18 um ca. 90° ist das Verbindungselement 20 derart ausgestaltet, dass es den Spannbolzenkopf 24 weg von der Klemmeinrichtung 5 bewegt. Dabei wird die Klemmeinrichtung 5 auf den Kraftüberträger 7 gedrückt und der Kraftüberträger 7 wiederum drückt gegen die Federkraft der Federelemente in den Federtaschen 8, 14 und 9, 15 gegen die Klemmbacken 1, 2, die derart gestaltet sind, dass nun der Freibereich der wannenförmigen Ausnehmung 23 verringert wird.

Die Verringerung des Freibereichs der wannenförmigen Ausnehmung 23 ist derart hoch, als dass ein in die wannenförmige Ausnehmung 23 eingebrachter Arm festgeklemmt wird und die erfindungsgemässe Klammer an dem Arm nicht mehr hin und her verschoben werden kann. In gleicher Weise ist es dann auch nicht mehr möglich, dass die erfindungsgemässe Klammer von dem Arm abgezogen werden kann.

Eine entsprechende Entspannung findet erst dann statt, wenn der Griff 18 entweder wieder in die ursprüngliche Position zurückgebracht wird, oder um weitere 90° hin zu der Seite der Klemmbacke 1 in den Figuren 1 und 2 geschwenkt wird. In einer solchen Position bildet der Griff 18 in etwa eine rechtwinklige Verlängerung des Spannbolzens 3. Bei einem angespannten Zustand bildet der Griff 18 die geradlinige Verlängerung des Spannbolzens 3.

**Bezugszeichenliste**

| | | | | | |
|---|---|---|---|---|---|
| 1 | Klemmbacke | 34 | | 67 | |
| 2 | Klemmbacke | 35 | | 68 | |
| 3 | Spannbolzen | 36 | | 69 | |
| 4 | Spannhebel | 37 | | 70 | |
| 5 | Klemmeinrichtung | 38 | | 71 | |
| 6 | Klemmbackenaufnahme | 39 | | 72 | |
| 7 | Kraftüberträger | 40 | | 73 | |
| 8 | Federtasche | 41 | | 74 | |
| 9 | Federtasche | 42 | | 75 | |
| 10 | Verjüngung | 43 | | 76 | |
| 11 | Aufnahme | 44 | | 77 | |
| 12 | Drehpunkt | 45 | | 78 | |
| 13 | Drehpunkt | 46 | | 79 | |
| 14 | Federtasche | 47 | | | |
| 15 | Federtasche | 48 | | | |
| 16 | Öffnung | 49 | | | |
| 17 | Drehachse | 50 | | | |
| 18 | Griff | 51 | | | |
| 19 | Halteknauf | 52 | | | |
| 20 | Verbindungselement | 53 | | | |
| 21 | Anschlagpunkt | 54 | | | |
| 22 | Anschlaqpunkt | 55 | | | |
| 23 | w. Ausnehmung | 56 | | | |
| 24 | Spannbolzenkopf | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Klammer zur Anbringung an einem Arm einer Wundspreizvorrichtung mit zwei Klemmbacken (1, 2) und einem Spannbolzen (3) mit einem einends des Spannbolzens (3) angebrachten endseitigen Spannbolzenkopf (24), sowie einer andernends des Spannbolzens (3) angeordneten wannenförmigen Ausnehmung (23) zur Aufnahme des Arms der Wundspreizvorrichtung, die in der Verlängerung des Ausnehmung (23) angeordnet ist, wobei sich die eine Klemmbacke (1) einerseits andernends des Spannbolzens (3) und die andere Klemmbacke (2) andererseits andernends des Spannbolzens (3) befindet, wobei der Spannbolzen (3) eine Klemmbackenaufnahme (6) umfasst, wobei ein Kraftüberträger (7) an dem Spannbolzen (3) gleitbar angeordnet ist
**dadurch gekennzeichnet, dass**
die eine Klemmbacke (1) über einen Drehpunkt (13) und die andere Klemmbacke (2) über einen Drehpunkt (12) mit der Klemmbackenaufnahme (6) verbunden ist, wobei die eine Klemmbacke (1) und die andere Klemmbacke (2) jeweils drehbar an der Klemmbackenaufnahme (6) gelagert sind, wobei der Kraftüberträger (7) gegenüber den zumindest zwei Klemmbacken (1, 2) über jeweils ein Federelement gelagert ist, wobei eine Klemmeinrichtung (5) auf den Kraftüberträger (7) drückbar ist und der Kraftüberträger (7) wiederum gegen die Federkraft der Federelemente in Federtaschen (8, 14 und 9, 15) gegen die Klemmbacken (1, 2) drückbar ist.

2. Klammer nach Anspruch 1, **dadurch gekennzeichnet, dass** eine konische Verjüngung (10) des Kraftüberträgers (7) andernseits der zumindest zwei Klemmbacken (1, 2) angeordnet ist, welche in eine Aufnahme (11) einer Klemmeinrichtung (5) anschlagend verschieblich aufgenommen ist.

3. Klammer nach einem der vorigen Ansprüche, **dadurch gekennzeichnet, dass** der Spannbolzen (3) einen Spannhebel (4) umfasst.

4. Klammer nach einem der Ansprüche 1 bis 3, **dadurch gekennzeichnet, dass** die eine Klemmbacke (1) einerseits der wannenförmigen Ausnehmung (23) und die andere Klemmbacke (2) andererseits der wannenförmigen Ausnehmung (23) angeordnet sind.

5. Klammer nach einem der Ansprüche 2-4 sofern abhängig von Anspruch 2, **dadurch gekennzeichnet, dass** der Kraftüberträger (7) andernseits der Klemmbacken (1, 2) mit der Klemmeinrichtung (5) in Wirkverbindung steht.

6. Klammer nach Anspruch 5, **dadurch gekennzeichnet, dass** die Klemmeinrichtung (5) mit dem Spannhebel (4) in Wirkverbindung steht, wobei der Spannhebel (4) einen gemeinsamen Drehpunkt mit dem Spannbolzen (3) aufweist.

## Claims

1. A brace for mounting on an arm of a wound retracting device, having two clamping jaws (1, 2) and a tensioning bolt (3), which has a terminal tensioning bolt head (24) that is mounted at one end of the tensioning bolt (3) and a trough-shaped recess (23) that is arranged at the second end of the tensioning bolt (3), for receiving the arm of the wound retracting device, which is arranged in the prolongation of the recess (23), wherein the one clamping jaw (1) is located on one side at the second end of the tensioning bolt (3) and the second clamping jaw (2) is located on the other side at the second end of the tensioning bolt (3), wherein the tensioning bolt (3) comprises a clamping jaw receiver (6), wherein a force-transmitting element (7) is arranged slidably on the tensioning bolt (3),
**characterised in that**
the one clamping jaw (1) is connected to the clamping jaw receiver (6) by way of a pivot point (13) and the second clamping jaw (2) is connected to the clamping jaw receiver (6) by way of a pivot point (12), wherein the one clamping jaw (1) and the second clamping jaw (2) are each borne pivotally on the clamping jaw receiver (6), wherein the force-transmitting element (7) is borne opposite the at least two clamping jaws (1, 2) by way of a respective spring element, wherein a clamping device (5) may be pressed onto the force-transmitting element (7) and the force-transmitting element (7) may in turn be pressed, in opposition to the spring force of the spring elements in spring pockets (8, 14 and 9, 15), against the clamping jaws (1, 2).

2. A brace according to Claim 1, **characterised in that** a conical tapering (10) of the force-transmitting element (7) is arranged on the other side from the at least two clamping jaws (1, 2) and is received displaceably and in abutting manner in a receiver (11) of a clamping device (5).

3. A brace according to one of the preceding clams, **characterised in that** the tensioning bolt (3) comprises a tensioning lever (4).

4. A brace according to one of Claims 1 to 3, **characterised in that** the one clamping jaw (1) is arranged on one side of the trough-shaped recess (23) and the second clamping jaw (2) is arranged on the other side of the trough-shaped recess (23).

5. A brace according to one of Claims 2 - 4 where they are dependent from Claim 2, **characterised in that** the force-transmitting element (7) is in operative connection with the clamping device (5) on the other side from the clamping jaws (1, 2).

6. A brace according to Claim 5, **characterised in that** the clamping device (5) is in operative connection with the tensioning lever (4), wherein the tensioning lever (4) has a common pivot point with the tensioning bolt (3).

## Revendications

1. Pince à placer sur un bras d'un dispositif de rétracteur chirurgical avec deux mâchoires de serrage (1, 2) et un boulon de serrage (3) avec une tête de boulon de serrage (24) du côté d'extrémité placée à une extrémité du boulon de serrage (3), ainsi qu'un évidement en forme de cuvette (23) disposé à l'autre extrémité du boulon de serrage (3), destiné à recevoir le bras du dispositif de rétracteur chirurgical disposé dans le prolongement de l'évidement (23), l'une mâchoire de serrage (1) se trouvant d'un côté à l'autre extrémité du boulon de serrage (3) et l'autre mâchoire de serrage (2) se trouvant de l'autre côté à l'autre extrémité du boulon de serrage (3), le boulon de serrage (3) comportant un moyen de réception de mâchoire de serrage (6), un transmetteur de force (7) étant disposé de manière coulissante sur le boulon de serrage (3),
**caractérisée par le fait que**
l'une mâchoire de serrage (1) est connectée, par l'intermédiaire d'un pivot (13), et l'autre mâchoire de serrage (2) est connectée, par l'intermédiaire d'un pivot (12), au moyen de réception de mâchoire de serrage (6), l'une mâchoire de serrage (1) et l'autre mâchoire de serrage (2) étant montées en rotation, chacune, sur le moyen de réception de mâchoire de serrage (6), le transmetteur de force (7) étant monté vis-à-vis de chacune des au moins deux mâchoires de serrage (1, 2) par l'intermédiaire d'un élément de ressort, un dispositif de serrage (5) pouvant être poussé contre le transmetteur de force (7) et le transmetteur de force (7) pouvant, à son tour, être poussé, à l'encontre de la force de ressort des éléments de ressort dans des poches à ressort (8, 14 et 9, 15), contre les mâchoires de serrage (1, 2).

2. Pince selon la revendication 1, **caractérisée par le fait qu'**un rétrécissement conique (10) du transmetteur de force (7) est disposé de l'autre côté des au moins deux mâchoires de serrage (1, 2), lequel est reçu déplaçable en butée dans un moyen de réception (11) d'un dispositif de serrage (5).

3. Pince selon l'une quelconque des revendications précédentes, **caractérisée par le fait que** le boulon de serrage (3) comprend un levier de serrage (4).

4. Pince selon l'une des revendications 1 à 3, **caractérisée par le fait que** l'une mâchoire de serrage (1) est disposée d'un côté de l'évidement en forme de cuvette (23) et l'autre mâchoire de serrage (2) est disposée de l'autre côté de l'évidement en forme de cuvette (23).

5. Pince selon l'une quelconque des revendications 2 à 4, pour autant qu'elle soit dépendante de la revendication 2, **caractérisée par le fait que** le transmetteur de force (7) de l'autre côté des mâchoires de serrage (1, 2) se trouve en liaison de fonctionnement avec le dispositif de serrage (5).

6. Pince selon la revendication 5, **caractérisée par le fait que** le dispositif de serrage (5) se trouve en liaison de fonctionnement avec le levier de serrage (4), le levier de serrage (4) présentant un pivot commun avec le boulon de serrage (3).
